# EUROPEAN PATENT APPLICATION

(11) **EP 1 628 134 A1**
(43) Date of publication of application: **22.02.2006**
(21) Application number: 04745293.3
(22) Date of filing: 25.05.2004
(51) Int. Cl.: G01N 33/48, G01N 33/72, G01N 33/66

(54) **METHOD OF STABILIZING SAMPLE**

(30) Priority: 28.05.2003 JP 2003151418
(71) Applicant: Arkray, Inc., Kyoto-shi, Kyoto 601-8045 (JP)
(72) Inventor: HIRAI, Kaoru, c/o Arkray, Inc., Kyoto-shi, Kyoto 601-8045 (JP); YONEHARA, Satoshi, c/o Arkray, Inc., Kyoto-shi, Kyoto 601-8045 (JP)
(74) Representative: Webber, Philip Michael
(86) International application number: PCT/JP2004/007084
(87) International publication number: WO 2004/106919

(57) **Abstract**

A method for stabilizing a hemolyzed sample that does not affect an accuracy of determination in a measurement even after the sample is left standing at room temperature, and a hemolysis reagent solution used therefor are provided. Using a hemolysis reagent solution in which a surfactant is contained and carbon dioxide is dissolved, blood cells are hemolyzed so as to prepare a hemolyzed sample. Even after the thus prepared hemolyzed sample is stored at room temperature, for example, glycated proteins or the like can be measured with an accuracy of determination similar to that for the hemolyzed sample immediately after preparation. The concentration of the carbon dioxide dissolved in the hemolyzed sample at 2°C to 35°C is at least 8 mmol/L.

## Description

### Technical Field

The present invention relates to a method for stabilizing hemolyzed samples.

### Background Art

Among glycated proteins in blood cells, glycated hemoglobin (HbA_{1c}), in particular, is a significant indicator for the diagnosis, therapy and the like of diabetes because it reflects the patient's past history of blood glucose levels.

Glycated hemoglobin is measured as a glycation ratio of hemoglobin by, for example, a high performance liquid chromatography (HPLC), a minicolumn method, an immunoassay, etc. Recently, a measurement conducted by an enzymatic method using glycated amino acid oxidoreductase (FAOD) also has been developed.

In the above-noted enzymatic method, first, blood cells are hemolyzed to prepare a hemolyzed sample. Afructosyl amino acid oxidase (hereinafter, referred to as "FAOD") is added to this hemolyzed sample and then allowed to act on a glycated portion of the glycated hemoglobin, thus generating hydrogen peroxide. The amount of this hydrogen peroxide corresponds to the glycation amount of the glycated hemoglobin. Then, a peroxidase (hereinafter, referred to as "POD") and a reducing agent further are added to this sample, so that a redox reaction occurs between the hydrogen peroxide and the reducing agent with the POD as a catalyst. At this time, if a substrate that develops color by oxidation is used as the reducing agent, the amount of the hydrogen peroxide can be determined by measuring the color developed. As a result, the amount of the glycated hemoglobin in blood cells can be determined. Such an enzymatic method is applied in the field of clinical medicine in practice.

### Disclosure of Invention

However, this enzymatic method has the following problem. Although a hemolyzed sample has to be prepared first as described above when measuring glycated proteins, the glycated proteins are not always measured immediately after preparing the hemolyzed sample. In other words, it is not until a certain number of the hemolyzed samples are prepared that these hemolyzed samples are all measured at one time. Therefore, each of the prepared hemolyzed samples is left for a different period of time until it is measured, and since the hemolyzed samples are left standing as above, there has been a phenomenon of lowered accuracy of determination. More specifically, even when the same hemolyzed samples are left standing at room temperature, the measurement values thereof increase over time depending on how long each is left standing, leading to the problem that accurate values of the amount of glycated proteins cannot be obtained. In order to avoid such a problem and maintain an excellent accuracy of determination, it is required to measure the glycated proteins immediately after preparing the hemolyzed samples. However, in the field of clinical medicine, a lot of specimens need to be treated, so that it also is practically difficult to make measurements immediately after preparing the hemolyzed samples of individual specimens in view of efficiency.

Accordingly, it is an object of the present invention to provide a method for stabilizing a hemolyzed sample that does not affect an accuracy of determination in a measurement even after the sample is left standing at room temperature, for example.

In order to achieve the above-mentioned object, a method for stabilizing a hemolyzed sample according to the present invention includes causing a hemolyzed sample obtained by hemolyzing blood cells to contain at least one selected from the group consisting of a carbonate ion, a bicarbonate ion and carbon dioxide.

The inventors of the present invention conducted a keen study to determine the reason why the measurement value of glycated hemoglobin increased overtime as described above when the hemolyzed sample was left standing at room temperature. Then, the inventors speculated that, since carbon dioxide in the air dissolved in the hemolyzed sample while the hemolyzed sample was left standing, the dissolved carbon dioxide gradually changed a hemoglobin structure in this sample, causing a change in a digestive reaction speed by protease and a change in a redox capability of hemoglobin itself, so that the above-described redox reaction by FAOD and POD was affected, resulting in a higher measurement value. As a result of the study based on such a speculation, the inventors found that, by causing carbon dioxide to dissolve in a hemolyzed sample or by causing a hemolyzed sample to contain a carbonate ion or a bicarbonate ion, it is possible to suppress the above-described variation of the measurement value over time caused by leaving the hemolyzed sample to stand, and arrived at the present invention. Incidentally, the inventors of the present invention found for the first time that the measurement value increased over time when the hemolyzed sample was left standing, that this increase was assumed to be attributable to the dissolution of carbon dioxide and that the above-described problem could be solved by causing the hemolyzed sample to contain carbon dioxide, a carbonate ion or a bicarbonate ion in advance. According to such a method for stabilizing a hemolyzed sample, it is not necessary to make measurements by enzyme reaction immediately after preparing the hemolyzed sample. Consequently, this is an extremely useful method in that glycated proteins including glycated hemoglobin can be measured very efficiently in the clinical field as described above.

Further, as described above, the method for stabilizing a hemolyzed sample according to the present invention can be carried out using a hemolysis reagent solution for hemolyzing a blood cell containing a surfactant and further containing at least one selected from the group consisting of a carbonate ion, a bicarbonate ion and carbon dioxide. Such a hemolysis reagent solution makes it easy to stabilize a hemolyzed sample according to the present invention as described above and thus is a reagent that is very useful at the time of measuring glycated proteins in a clinical field or the like, for example. Moreover, by the method for stabilizing a hemolyzed sample according to the present invention, a hemolyzed sample can be produced.

### Brief Description of Drawings

[FIG. 1] FIG. 1 is a graph showing a relationship between a storage period and a variation in a measurement value of a hemolyzed sample in an example of the hemolyzed sample prepared according to the present invention.
[FIG. 2] FIG. 2 is a graph showing a relationship between a storage period and a variation in a measurement value of another hemolyzed sample in the above-noted example.
[FIG. 3] FIG. 3 is a graph showing a relationship between a storage period and a variation in a measurement value of yet another hemolyzed sample in the above-noted example.

### Description of the Invention

As described above, a method for stabilizing a hemolyzed sample according to the present invention includes causing a hemolyzed sample obtained by hemolyzing blood cells to contain at least one selected from the group consisting of a carbonate ion, a bicarbonate ion and carbon dioxide. Incidentally, causing the hemolyzed sample to contain carbon dioxide in the present invention refers to causing carbon dioxide to dissolve in the hemolyzed sample.

First, an exemplary method for causing carbon dioxide to dissolve in a hemolyzed sample so as to stabilize the hemolyzed sample will be described.

The concentration of the carbon dioxide dissolved in the hemolyzed sample can be determined suitably according to, for example, an amount of blood cells subjected to a hemolysis treatment and, in the case of 2°C to 35°C, for example, is at least about 8 mmol/L, preferably ranges from about 10 to 80 mmol/L and more preferably ranges from about 15 to 80 mmol/L. More specifically, in the case where the concentration of blood cells in the hemolyzed sample is about 5 g/L, the concentration of the carbon dioxide dissolved at about 2°C to 35°C preferably is set to at least about 8 mmol/L, for example, more preferably ranges from about 10 to 80 mmol/L and particularly preferably ranges from about 15 to 80 mmol/L. Incidentally, the concentration of carbon dioxide in a hemolyzed sample that is not intentionally caused to contain carbon dioxide (the blood cell concentration: 5 g/L) usually is about 0.4 to 1 mmol/L.

In the present invention, it is preferable further to cause the hemolyzed sample to contain a surfactant. By causing it to contain the surfactant, a protease treatment for degrading glycated proteins such as glycated hemoglobin can be carried out efficiently when measuring the glycated proteins, for example. Further, although carbon dioxide may have an influence to change, for example, the digestive speed of hemoglobin as described above, the addition of the surfactant accelerates the digestive reaction. The surfactant is not particularly limited and can be, for example, nonionic surfactants such as polyoxyethylene-p-t-octylphenyl ether (e.g. Triton series surfactants), polyoxyethylene sorbitan alkyl ester (e.g. Tween series surfactants), polyoxyethylene alkyl ether (e.g. Brij series surfactants) and the like. Specific examples thereof are Triton X-100, Tween-20, Brij 35, etc.

For example, the concentration of the surfactant contained in the hemolyzed sample ranges preferably from about 6 to 40 mmol/L, more preferably from about 10 to 40 mmol/L and particularly preferably from about 15 to 40 mmol/L. More specifically, in the case where the concentration of blood cells in the hemolyzed sample is about 5 g/L, the concentration of the surfactant at about 2°C to 35°C is at least about 8 mmol/L, for example, preferably ranges from about 10 to 40 mmol/L and more preferably ranges from about 15 to 40 mmol/L.

In order to cause carbon dioxide to dissolve in the hemolyzed sample, it is preferable that the stabilizing method of the present invention further includes a process of hemolyzing blood cells using a hemolysis reagent solution in which the surfactant is contained and the carbon dioxide is dissolved, for example. By hemolyzing blood cells using such a hemolysis reagent solution, it is possible to prepare a hemolyzed sample in which carbon dioxide is dissolved.

The blood cells may be hemolyzed by, for example, adding the above-noted hemolysis reagent solution to whole blood or adding and mixing the above-noted hemolysis reagent solution into blood cells (pellets) collected from whole blood. The blood cells can be collected by a conventionally known method, for example, centrifugation, a treatment with a membrane such as a blood cell separation membrane, or the like.

The ratio (the mole ratio A: B) between the surfactant (A) and the carbon dioxide (B) in the hemolysis reagent solution is not particularly limited but ranges preferably from 1 : 0.5 to 1 : 13, more preferably from 1 : 2 to 1 : 13 and particularly preferably from 1 : 2 to 1 : 3.

The concentrations of the surfactant and the carbon dioxide in the hemolysis reagent solution can be determined suitably according to, for example, the added amount to blood cells or the amount of blood cells and are, for example, surfactant: about 6 to 42 mmol/L and carbon dioxide: about 8 to 84 mmol/L, preferably surfactant: about 6.3 to 42 mmol/L and carbon dioxide: about 8.4 to 84 mmol/L, more preferably surfactant: 10 to 40 mmol/L and carbon dioxide: 10 to 80 mmol/L, and particularly preferably surfactant: 15 to 40 mmol/L and carbon dioxide: 15 to 80 mmol/L. It should be noted that the above-mentioned concentration of carbon dioxide refers to the concentration at 2°C to 35°C, for example.

The above-noted hemolysis reagent solution in which the surfactant is contained and the carbon dioxide is dissolved can be prepared by, for example, bubbling a 99.9% carbon dioxide gas through a solution containing the surfactant. The bubbling can be carried out using, for example, a carbon dioxide gas cylinder or the like. Although the condition thereof is not particularly limited, the bubbling usually is conducted at 2°C to 35°C. More specifically, in the case of 10 mL of the above-noted solution, the bubbling is conducted, for example, at a natural atmospheric pressure at 1.5°C to 35°C for 10 seconds, thereby obtaining a hemolysis reagent solution whose concentration of dissolved carbon dioxide is about 20 to 80 mmol/L.

A solvent in the solution containing the surfactant is not particularly limited and can be, for example, water or a buffer of various kinds. Further, the carbon dioxide may be dissolved in advance in the solvent for preparing the solution, for example, instead of being dissolved in the solution containing the surfactant as described above.

The mixture ratio of blood cells and the hemolysis reagent solution is not particularly limited but preferably is set so that the concentration of dissolved carbon dioxide and the concentration of the surfactant in the prepared hemolyzed sample are in the range described above, for example.

Moreover, in the stabilizing method according to the present invention, for causing the carbon dioxide to dissolve in the hemolyzed sample, it also may be possible to prepare a hemolyzed sample by a method using a surfactant, a method using ultrasonic waves, a method utilizing a difference in osmotic pressure or the like, followed by bubbling a carbon dioxide gas directly through this hemolyzed sample, for example, instead of using the above-described hemolysis reagent solution.

Next, an exemplary method for causing a hemolyzed sample to contain a carbonate ion or a bicarbonate ion so as to stabilize the hemolyzed sample will be described.

The concentration of the carbonate ion or the bicarbonate ion in the hemolyzed sample can be determined suitably according to, for example, an amount of blood cells subjected to a hemolysis treatment and, for example, is at least about 8 mmol/L, preferably ranges from about 10 to 80 mmol/L and more preferably ranges from about 15 to 80 mmol/L. More specifically, in the case where the concentration of blood cells in the hemolyzed sample is 5 g/L, the concentration of the carbonate ion or the bicarbonate ion is at least about 8 mmol/L, for example, preferably ranges from about 10 to 80 mmol/L and more preferably ranges from about 15 to 80 mmol/L. Incidentally, one or both of the carbonate ion and the bicarbonate ion may be contained. Also, in the case where both of the ions are contained, the total concentration thereof may be the above-mentioned concentration (e.g., at least 8 mmol/L), for example.

Similarly to the above, it is preferable further to cause the hemolyzed sample to contain a surfactant. The kind and concentration of the surfactant are not particularly limited and, for example, are similar to the above.

In order to cause the hemolyzed sample to contain the carbonate ion or the bicarbonate ion, it is preferable that the present invention includes a process of hemolyzing blood cells using a hemolysis reagent solution in which the surfactant is contained and the carbonate ion or the bicarbonate ion is contained, for example. By hemolyzing blood cells using such a hemolysis reagent solution, it is possible to prepare a hemolyzed sample containing the carbonate ion or the bicarbonate ion.

The hemolysis reagent solution in which the surfactant is contained and the carbonate ion or the bicarbonate ion is dissolved can be prepared by, for example, dissolving a carbonate in a solution containing a surfactant.

The above-noted carbonate preferably is an alkali metal salt, for example. More specifically, it is possible to use sodium carbonate, potassium carbonate, magnesium carbonate, lithium carbonate, potassium sodium carbonate, potassium bicarbonate, sodium bicarbonate, ammonium carbonate, ammonium bicarbonate and Tris carbonate, and among them, sodium carbonate is preferable. Also, they may be used alone or in combinations of two or more kinds.

The addition ratio (mole ratio A : C) between the surfactant (A) and the carbonate (C) in the hemolysis reagent solution preferably ranges from 1 : 0.2 to 1 : 27, for example, more preferably ranges from 1 : 1.5 to 1 : 27 and particularly preferably ranges from 1 : 1.5 to 1 : 4.

The concentration of the carbonate ion or the bicarbonate ion in the hemolysis reagent solution can be determined suitably according to, for example, the added amount to blood cells or the amount of blood cells and, for example, is at least about 8 mmol/L, preferably is at least about 8.4 mmol/L, more preferably ranges from about 10 to 80 mmol/L and particularly preferably ranges from about 15 to 80 mmol/L.

Moreover, for causing the hemolyzed sample to contain the carbonate ion or the bicarbonate ion, it may be possible to prepare a hemolyzed sample and then dissolve the above-noted carbonate or add and mix a solution in which the carbonate is dissolved directly into this hemolyzed sample, for example, instead of using the above-described hemolysis reagent solution.

As a conventional problem, in particular, blood is left standing while being hemolyzed, so that the value of HbA_{1c} rises as described above. Accordingly, in the stabilizing method according to the present invention, it is appropriate that the carbon dioxide, the carbonate ion or the bicarbonate ion be contained at least when the hemolyzed sample is left standing. In the stabilizing method according to the present invention, the carbon dioxide, the carbonate ion or the bicarbonate ion may be contained in the hemolyzed sample as described above or contained in advance in a pre-hemolyzed specimen, for example. Further, it also may be possible to cause the hemolyzed sample or the pre-hemolyzed specimen to contain them directly or to cause the hemolysis reagent solution to contain the carbon dioxide, the carbonate ion or the bicarbonate ion as described above and then add this reagent solution to the pre-hemolyzed specimen. Among the above, in view of convenience and simplicity at the site of clinical tests, for example, it is preferable to cause the hemolysis reagent to contain the carbon dioxide, the carbonate ion or the bicarbonate ion in advance and then add this reagent to the pre-hemolyzed specimen.

The thus prepared hemolyzed sample in which the carbon dioxide, the carbonate ion or the bicarbonate ion is contained can maintain a stable state for a long period in the case where it is stored at room temperature, for example. Therefore, for example, even when measuring glycated proteins in the hemolyzed sample by the enzyme reaction after storage as described above, variation in the measurement value over time can be suppressed. Although the present invention is applicable to the case of storing hemolyzed samples at a low temperature, for example, it is considered useful when storing hemolyzed samples at room temperature (for example, about 15°C to 35°C) because the storage problem under room temperature condition can be solved as described above. In particular, in the field of analytical chemistry in clinical medicine, etc. described above, prepared hemolyzed samples often are stored at room temperature in practice. Considering the fact that the above-described problem arises due to the storage under such conditions, the present invention is useful for stabilization at room temperature. Further, although it of course is possible to measure the hemolyzed samples immediately after preparation, it is preferable that the present invention is applied to the case in which the use immediately after preparation is difficult in the field of clinical medicine as described above because the present invention can avoid the problem at the time of storage as mentioned above. In accordance with the present invention, a measurement value can be maintained at approximately the same level as that immediately after preparation even after about 26 hours since the hemolyzed sample is prepared, and a preferable storage period is about 3 to 10 hours.

The hemolyzed sample prepared by the present invention can be used as a sample for measuring, for example, glycated hemoglobin, hemoglobin, glycoalbumin, albumin, globulin, myoglobin or the like, and glycated hemoglobin is particularly preferable.

The following is a description of an exemplary method for measuring glycated hemoglobin in a hemolyzed sample prepared according to the present invention.

First, the hemolyzed sample is subjected to a protease treatment so as to degrade glycated hemoglobin in the sample. The protease treatment usually is carried out in a buffer. The treatment condition is determined suitably according to the kind of protease used, the kind and concentration of glycated hemoglobin, etc.

The protease is not particularly limited but can be, for example, serine proteases, thiol proteases, metalloproteinases or the like. More specifically, it is preferable to use trypsin, proteinase K, chymotrypsin, papain, bromelain, subtilisin, elastase, aminopeptidase and the like. In the case where the glycated protein to be degraded is glycated hemoglobin, the protease preferably is a protease degrading the glycated hemoglobin selectively, e.g., bromelain, papain, trypsin derived from porcine pancreas, metalloproteinase and protease derived from *Bacillus subtilis*. Examples of the protease derived from *Bacillus subtilis* include trade name Protease N (manufactured by Fluka Chemie AG, for example), trade name Protease N "AMANO" (manufactured by Amano Enzyme Inc.) and the like. Examples of the metalloproteinase include metalloproteinase (EC 3. 4. 24. 4) derived from the genus *Bacillus* (for example, trade name Toyoteam, manufactured by TOYOBO CO., LTD.) and the like. Among these, metalloproteinase, bromelain and papain are more preferable, and metalloproteinase is most preferable. By using the protease that allows a selective degradation as above, a degradation product of a specific protein can be prepared selectively.

The buffer can be, for example, a CHES buffer, a CAPSO buffer, a CAPS buffer, a phosphate buffer, a Tris buffer, an EPPS buffer, a HEPES buffer or the like. The pH thereof ranges, for example, from 6 to 13 and preferably from 7 to 11. Further, the final concentration of the buffer in the protease treatment solution ranges, for example, from 1.0 to 10 mmol/L.

More specifically, when the above-described hemolyzed sample is treated using metalloproteinase as the protease, usually, the concentration of the metalloproteinase in the reaction solution ranges from 0.1 to 40 MU/L, the concentration of blood cells in the reaction solution ranges from 0.05 to 15 vol%, a reaction temperature ranges from 15°C to 37°C, a reaction period ranges from 1 minute to 24 hours, and a pH ranges from 6 to 12.

Also, when the above-described hemolyzed sample is treated using protease K as the protease, usually, the concentration of the protease in the reaction solution ranges from 10 to 300 KU/L, the concentration of blood cells in the reaction solution ranges from 0.05 to 15 vol%, a reaction temperature ranges from 15°C to 37°C, a reaction period ranges from 1 minute to 24 hours, and a pH ranges from 6 to 12. In addition, there is no particular limitation on the kind of the buffer, and it is possible to use a Tris hydrochloric acid buffer, an EPPS buffer, a PIPES buffer or the like, for example.

Next, a glycated hemoglobin degradation product obtained by the protease treatment is treated with FAOD, which will be described later. This FAOD treatment catalyzes the reaction shown by Formula (1) below. The glycated portion of the glycated hemoglobin degradation product to be reacted with the FAOD varies depending on a catalytic reaction of FAOD used and preferably is a glycated amino group in a side chain of an amino-acid residue or a glycated α-amino group, for example. Among them, the glycated amino group in the side chain of the amino-acid residue is preferable because FAOD having a catalytic function described later acts easily thereon. Examples thereof include a glycated amino group in a side chain of a lysine residue and a glycated amino group in a side chain of an arginine residue.

It is preferable that the FAOD treatment is carried out in a buffer as in the above protease treatment. The buffer is not particularly limited but can be a buffer similar to that in the above protease treatment.

The conditions of this treatment are as follows: for example, the concentration of the FAOD in the reaction solution ranges from 200 to 30,000 U/L, the concentration of hemoglobin in the reaction solution ranges from 0.1 to 10 g/L, the reaction temperature ranges from 15°C to 37°C, the reaction period ranges from 1 to 20 minutes, and the pH ranges from 7 to 9.

Next, the hydrogen peroxide formed in the FAOD treatment is measured by a redox reaction using a POD and a substrate that develops color by oxidation.

This redox reaction by the POD usually is carried out in a buffer. The conditions thereof are determined suitably depending on the concentration of the hydrogen peroxide, etc. The conditions are usually as follows: the concentration of the POD in the reaction solution is 1 to 20000 IU/L, the concentration of the substrate is 0.0001 to 1 mmol/L, the reaction temperature is 20°C to 37°C, the reaction period is 1 to 5 minutes, and the pH is 6 to 9. Moreover, the kind of the buffer is not particularly limited, and the buffers similar to those in the FAOD treatment can be used.

The above-noted substrate that develops color by oxidation is not particularly limited but can be, for example, N-(carboxymethylaminocarbonyl)-4,4'-bis(dimethylamino)diphenylamine sodium salt, orthophenylenediamine (OPD), a substrate of a combination of Trinder's reagent and 4-aminoantipyrine or the like. As the above-noted N-(carboxymethylaminocarbonyl)-4,4'-bis(dimethylamino)diphenylamine sodium salt, trade name DA-64 (manufactured by DOJINDO LABORATORIES) can be used, for example. The Trinder's reagent can be, for example, phenols, phenol derivatives, aniline derivatives, naphthols, naphthol derivatives, naphthylamine or naphthylamine derivatives. Other than the 4-aminoantipyrine noted above, it also is possible to use aminoantipyrine derivatives, vanillin diamine sulfonic acid, methyl benzothiazolinone hydrazone (MBTH), sulfonated methyl benzothiazolinone hydrazone (SMBTH) or the like. Among these color-developing substrates, N-(carboxymethylaminocarbonyl)-4,4'-bis(dimethylamino)diphenylamine sodium salt is particularly preferable.

When the substrate that develops color by oxidation is used, the concentration of the hydrogen peroxide can be determined by measuring the color developed (i.e. absorbance in the reaction solution) with a spectrophotometer. Then, using this concentration, the amount of the glycated hemoglobin in the sample can be determined.

As the FAOD, a FAOD catalyzing a reaction represented by Formula (1) below can be used, for example.

[Formula 1] R¹-CO-CH₂-NH-R² + H₂O + O₂ → R¹-CO-CHO + NH₂-R² + H₂O₂ (1)

In Formula (1), R¹-CO-CH₂-NH-R² represents a glycated protein, a glycated peptide and a glycated amino acid, for example. In Formula (1), R¹ represents a hydroxyl group or a residue derived from the sugar before glycation (i.e., sugar residue). The sugar residue (R¹) is an aldose residue when the sugar before glycation is aldose, and is a ketose residue when the sugar before glycation is ketose. For example, when the sugar before glycation is glucose, it takes a fructose structure after glycation by an Amadori rearrangement. In this case, the sugar residue (R¹) becomes a glucose residue (an aldose residue). This sugar residue (R¹) can be represented, for example, by

-[CH(OH)]ₙ-CH₂OH

where n is an integer of 0 to 6. In Formula (1), R² is an amino acid residue or a peptide residue.

Although the catalytic reaction of the FAOD used is not particularly limited as long as it is a reaction as described above, it preferably is a catalytic reaction that acts on a glycated portion in which sugar bonds to an amino group other than the α-amino group in Formula (1) above. Further, the above-noted FAOD may have not only such a catalytic function but also a catalytic function of acting on the glycated portion in which sugar bonds to the α-amino group.

### Examples

### (Example 1)

### (Preparation of Standard Sample)

Lyophilized hemoglobin was dissolved in a hemolysis reagent solution A, which will be described below, thereby preparing a sample solution with a hemoglobin concentration of 4.1 g/L and an HbA_{1c} value of 4.4% (hereinafter, referred to as "sample Low") and a sample solution with a hemoglobin concentration of 5.3 g/L and an HbA_{1c} value of 10.4% (hereinafter, referred to as "sample High").

### (Preparation of Specimen Sample)

Human whole blood was subjected to a centrifugation treatment (3000 rpm, 10 minutes, 20°C) so as to collect erythrocytes. Hemolysis was carried out by adding and blending to these erythrocytes 50 times as much (by volume) hemolysis reagent solution A, which will be described below, thus preparing a specimen sample.

**(Hemolysis reagent solution A)**

| | |
|---|---|
| Surfactant (polyoxyethylene lauryl ether) | 12 g/L |
| CHES/MOPS buffer (pH 9.4) | CHES 80 mM |
| | MOPS 30 mM |
| Na₂CO₃ | 33 mM |

Each of the above-mentioned samples (hemolyzed samples) was left standing at 25°C for predetermined periods (0, 2, 4, 6 and 8 hours). Thereafter, 180 µL of a protease reagent, which will be described below, was added to 20 µL of the hemolyzed sample that had been left standing, treated at 37°C for 5 minutes, and 45 µL of a FAOD reagent, which will be described below, was added and treated at 37°C. Then, the absorbance in the reaction solution 5 minutes after adding the FAOD reagent was measured at a main wavelength of 700 nm and at a sub-wavelength of 570 nm. Further, the measurement value was substituted into a calibration curve showing the relationship between HbA_{1c} (%) and absorbance produced in advance using a standard substance of HbA_{1c}, thereby determining HbA_{lc} (%) of the hemolyzed sample. Then, the HbA_{1c} value (%) of each hemolyzed sample relative to the HbA_{1c} value (%) of the hemolyzed sample that was left standing for 0 hour was calculated, with the latter being "100%."

Additionally, a hemolysis reagent solution C was prepared, whose composition is the same as the above-described hemolysis reagent solution A except that the concentration of surfactant was 12 g/L and no sodium carbonate was added. Then, similarly to the above except that the hemolysis reagent solution A was replaced by the hemolysis reagent solution C, a hemolyzed sample was prepared, left standing and subjected to a color development reaction, and this served as Comparative example 1.

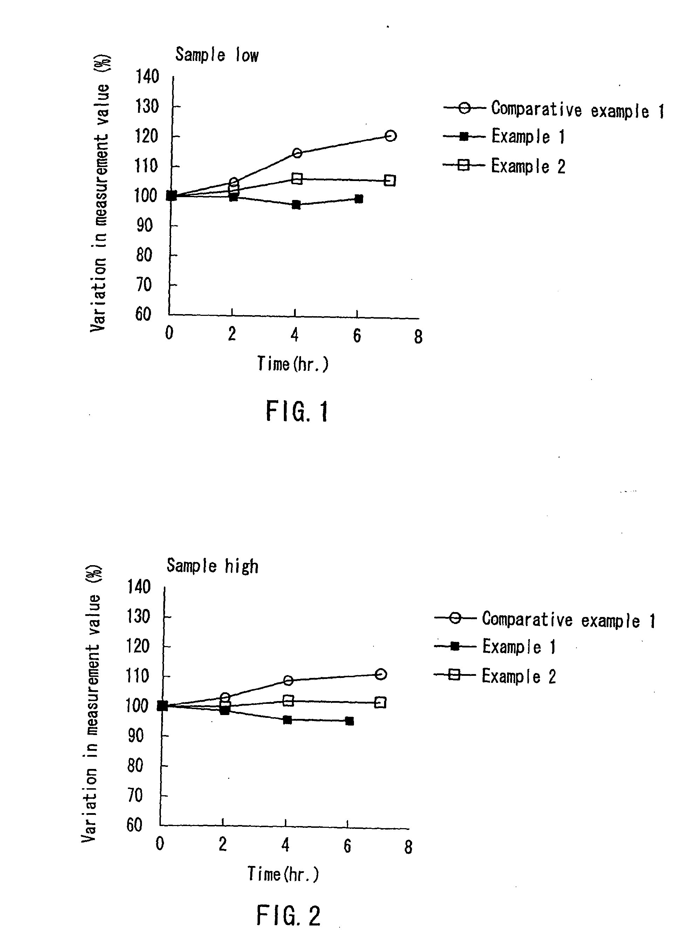

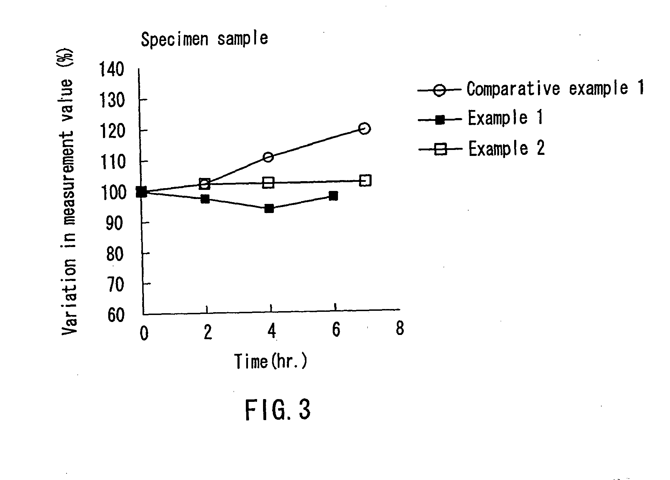

FIGs. 1 to 3 show the results. FIGs. 1 to 3 are graphs respectively showing the relationship between a period for which the hemolyzed sample was left standing and the relative HbA_{1c} value (%), with FIG. 1 showing the result of the sample Low, FIG. 2 showing the result of the sample High and FIG. 3 showing the result of the specimen sample.

**(Protease reagent)**

| | |
|---|---|
| Metalloprotease (manufactured by ARKRAY, INC.) | 2 MU/L |
| MES buffer (pH 5.5) (FAOD reagent) | 1 mM |
| FAOD (manufactured by ARKRAY, INC.) | 17.4 KU/L |
| POD (manufactured by Kikkoman Corporation) | 67.0 KU/L |
| Tris buffer (pH 7.0) | 300 mM |
| Trade name DA-64 (manufactured by DOJINDO LABORATORIES) | 71.3 µmol/L |

### (Example 2)

The absorbance was measured similarly to Example 1 described above except that the hemolysis reagent solution A was replaced by a hemolysis reagent solution B, which will be described later, thus determining the relative HbA_{1c} value. The results are shown also in FIGs. 1 to 3.

### (Hemolysis reagent solution B)

Using a hand-held gas cylinder (trade name: Standard Gas Co₂; manufactured by GL Sciences Inc.), bubbling of carbon dioxide was performed through a mixture solution with the same composition as the hemolysis reagent solution A except that no Na₂O₃ was added. The conditions of bubbling were at room temperature (about 20°C) at natural atmospheric pressure for 10 seconds. In this manner, a hemolysis reagent solution B whose carbon dioxide concentration was about 30 mmol/L was prepared.

As shown in FIGs. 1 to 3, according to Comparative example 1 using the hemolysis reagent solution C whose composition was the same as the hemolysis reagent solution A except that no sodium carbonate was added and no bubbling of carbon dioxide was performed, the relative HbA_{1c} value increased over time as the hemolyzed sample was left standing. In contrast, according to the Example using the hemolysis reagent solution A or the hemolysis reagent solution B, substantially no increase in the relative HbA_{1c} value was observed even after the hemolyzed samples were left standing for 7 hours similarly to Comparative example 1. From these results, it is clear that by causing carbon dioxide to dissolve in the hemolyzed sample in advance, an increase in the relative HbA_{1c} value as the hemolyzed sample was left standing can be prevented, so that a deterioration of the accuracy of determination as the hemolyzed sample was left standing can be prevented.

### (Example 3, Comparative example 2)

Furthermore, how the relative HbA_{1c} value varied in the case where the hemolyzed sample was left standing at 25°C for 24 hours was examined. First, a hemolyzed sample was prepared in a manner described above using the above-mentioned hemolysis reagent solution B. The hemolyzed sample immediately after preparation and the hemolyzed sample that had been left standing at 25°C for 24 hours were each subjected to the color development reaction, and the absorbance thereof was measured. Then, from a calibration curve showing the relationship between HbA_{1c} (%) and absorbance produced in advance using a standard substance of HbA_{1c}, HbA_{1c} (%) of each hemolyzed sample was determined. Also, as Comparative example 2, HbA_{1c} (%) was determined similarly to Example 1 except that the hemolysis reagent solution A was replaced by the hemolysis reagent solution C. Table 1 below shows the results. In the table below, 0 hr and 24 hr indicate periods for which the hemolyzed samples were left standing at 25°C, and the values inside parentheses indicate variations of HbA_{1c} (%) after 24 hours.

**[Table 1]**

| | Example 3 | | | Comparative example 2 | | |
|---|---|---|---|---|---|---|
| Sample | 0 hr | 24 hr | | 0 hr | 24 hr | |
| Sample Low | 5.5% | 5.8% | (0.3%) | 5.5% | 6.4% | (0.9%) |
| Sample High | 11.6% | 11.4% | (- 0.2%) | 11.6% | 12.5% | (0.9%) |

As shown in Table 1 above, the variation of measurement value was 0.9% according to Comparative example 2, whereas that was within the range of measurement error (about 0.5% or smaller) according to Example 3. Thus, it was found that a highly reliable HbA_{1c} concentration could be measured even after the hemolyzed samples were left standing.

### Industrial Applicability

As described above, in accordance with the present invention, even after a prepared hemolyzed sample is stored at room temperature, for example, glycated proteins can be measured with an accuracy of determination similar to that for the hemolyzed sample immediately after preparation. Therefore, the present invention is a method for stabilizing a hemolyzed sample that is useful especially in the case where a large amount of specimens have to be treated.

## Claims

1. A method for stabilizing a hemolyzed sample, comprising causing a hemolyzed sample to contain at least one selected from the group consisting of a carbonate ion, a bicarbonate ion and carbon dioxide.

2. The method according to claim 1, wherein a concentration of the carbon dioxide dissolved in the hemolyzed sample at 2°C to 35°C is at least 8 mmol/L.

3. The method according to claim 2, wherein the concentration of the carbon dioxide dissolved in the hemolyzed sample at 2°C to 35°C ranges from 8 to 80 mmol/L.

4. The method according to claim 1, wherein a concentration of the carbonate ion or the bicarbonate ion in the hemolyzed sample is at least 8 mmol/L.

5. The method according to claim 4, wherein the concentration of the carbonate ion or the bicarbonate ion in the hemolyzed sample ranges from 8 to 80 mmol/L.

6. The method according to claim 1, further comprising causing the hemolyzed sample to contain a surfactant.

7. The method according to claim 6, wherein a concentration of the surfactant in the hemolyzed sample ranges from 6 to 40 mmol/L.

8. The method according to claim 6, wherein a concentration of blood cells in the hemolyzed sample ranges from 3 to 7.5 wt%.

9. The method according to claim 1, comprising a process of hemolyzing blood cells using a hemolysis reagent solution containing a surfactant and containing at least one selected from the group consisting of the carbon dioxide, the carbonate ion and the bicarbonate ion.

10. The method according to claim 9, wherein the hemolysis reagent solution in which the carbon dioxide is dissolved is prepared by bubbling a carbon dioxide gas through a solution containing the surfactant.

11. The method according to claim 9, wherein a ratio (A : B) between the surfactant (A) and the carbon dioxide (B) in the hemolysis reagent solution ranges from 1 : 0.5 to 1 : 13.

12. The method according to claim 9, wherein the hemolysis reagent solution containing the carbonate ion is prepared by dissolving a carbonate in a solution containing the surfactant.

13. The method according to claim 12, wherein the carbonate is an alkali metal salt.

14. The method according to claim 13, wherein the alkali metal salt is at least one salt selected from the group consisting of sodium carbonate, potassium carbonate, magnesium carbonate, lithium carbonate, potassium sodium carbonate, potassium bicarbonate, sodium bicarbonate, ammonium carbonate, ammonium bicarbonate and Tris carbonate.

15. The method according to claim 9, wherein a ratio (A: C) between the surfactant (A) and a carbonate (C) ranges from 1 : 0.2 to 1 : 27.

16. The method according to claim 1, comprising bubbling a carbon dioxide gas through the hemolyzed sample.

17. The method according to claim 1, wherein a carbonate is dissolved in the hemolyzed sample.

18. The method according to claim 1, wherein the hemolyzed sample is used for measuring glycated hemoglobin.

19. A method for manufacturing a hemolyzed sample, comprising the method according to claim 1.

20. A hemolysis reagent solution for hemolyzing a blood cell comprising a surfactant, further comprising at least one selected from the group consisting of a carbonate ion, a bicarbonate ion and carbon dioxide.

21. The hemolysis reagent solution according to claim 20, wherein a concentration of the carbon dioxide dissolved at 2°C to 35°C is at least 8 mmol/L.

22. The hemolysis reagent solution according to claim 20, wherein a ratio (A : B) between the surfactant (A) and the carbon dioxide (B) ranges from 1 : 0.5 to 1 : 13.

23. The hemolysis reagent solution according to claim 20, wherein a concentration of the carbonate ion or the bicarbonate ion is at least 8 mmol/L.

24. The hemolysis reagent solution according to claim 20, wherein a ratio (A : C) between the surfactant (A) and a carbonate (C) ranges from 1 : 0.2 to 1 : 27.
